# EUROPÄISCHE PATENTANMELDUNG

(11) **EP 0 675 113 A2**
(43) Veröffentlichungstag der Anmeldung: **04.10.1995**
(21) Anmeldenummer: 95104384.3
(22) Anmeldetag: 24.03.1995
(51) Int. Cl.: C07D 239/50, C07D 239/545

(54) **Verfahren zur Herstellung von 5-Formylaminopyrimidinen**

(30) Priorität: 28.03.1994 DE 4410678
(71) Anmelder: SKW Trostberg Aktiengesellschaft, D-83308 Trostberg (DE)
(72) Erfinder: Thalhammer, Franz, Dr., D-83308 Trostberg (DE); Graefe, Jürgen, Dr., D-83308 Trostberg (DE)
(74) Vertreter: Huber, Bernhard, Dipl.-Chem.

(57) **Zusammenfassung**

Zur Herstellung von 5-Formylamino-pyrimidinen der allgemeinen Formel (I) 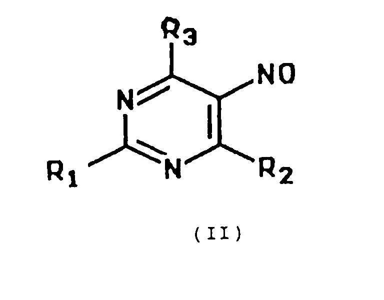
wobei die Reste R¹ bis R³ gleich oder verschieden sind und H, OH, SH, NH₂, Alkylamino, Halogen, O-Alkyl, S-Alkyl, Alkyl sowie Aryl bedeuten und Alkyl einen aliphatischen Rest mit 1 bis 4 Kohlenstoffatomen darstellt, aus den entsprechenden 5-Nitroso-pyrimidinen werden die Ausgangsverbindungen einer reduktiven Formylierung in Gegenwart eines Katalysators auf Edelmetallbasis sowie von Ameisensäure und von einem Formiat unterworfen. Auf diese Weise ist es möglich, mit geringem technischen Aufwand 5-Formylamino-pyrimidine mit einer Ausbeute von bis zu 98 % und einer Reinheit von mehr als 98 % herzustellen.

## Beschreibung

Gegenstand der Erfindung ist ein Verfahren zur Herstellung von 5-Formylamino-pyrimidinen aus den entsprechenden 5-Nitrosopyrimidinen durch reduktive Formylierung.

Hydrierungen substituierter 5-Nitrosopyrimidine nach verschiedenen Methoden sind bekannt. Üblicherweise wird als (groß)technisches Verfahren die Edelmetall-katalysierte Hydrierung mit Wasserstoff unter Druck verwendet. So wird beispielsweise gemäß der DE-PS 36 38 635 die katalytische Hydrierung von 2,4-Diamino-6-hydroxy-5-nitrosopyrimidin (DAHNP) und die Umsetzung des dabei erhaltenen 2,4,5-Triamino-6-hydroxy-pyrimidin (TAHP) mit Ameisensäure zu 2,4-Diamino-5-formylamino-6-hydroxy-pyrimidin (DAFHP) beschrieben, wobei die Reaktion in wäßriger Lösung bei Temperaturen zwischen 20 und 80°C und Wasserstoffdrücken zwischen 1,4 und 21 bar in Gegenwart bekannter Hydrierkatalysatoren durchgeführt wird und während der gesamten Reaktionszeit 0,8 bis 1,5 mol Base pro mol DAHNP kontinuierlich zugesetzt werden muß. Nachteilig bei dieser Reduktionsmethode ist einerseits der hohe Wasserstoffdruck, welcher den Einsatz teurer Autoklaven oder Schleifenreaktoren erforderlich macht, andererseits hat der Zusatz an äquimolaren Mengen Basen zum Lösen der Ausgangsverbindung einen zusätzlichen Salzanfall bei der Weiterverarbeitung der Reaktionslösungen zur Folge, wodurch die Umweltbelastung erhöht wird.

In der DE-OS 37 27 508 wird die Reduktion von 5-Nitrosopyrimidin-Derivaten mit Natriumdithionit und anschließende Formylierung zu den entsprechenden 5-Formylamino-pyrimidinen beschrieben. Diese Methode hat den Nachteil, daß neben der Freisetzung von SO₂ auch große Mengen an schwefelhaltigen Salzen als Nebenprodukte anfallen, so daß die für eine technische Produktion erforderliche Wirtschaftlichkeit nicht gegeben ist.

Der Erfindung liegt daher die Aufgabe zugrunde, ein Verfahren zur Herstellung von 5-Formylamino-pyrimidinen aus den entsprechenden 5-Nitrosopyrimidinen zu entwickeln, welches die genannten Nachteile des Standes der Technik nicht aufweist, sondern es erlaubt, in wirtschaftlich sinnvoller und umweltverträglicher Weise unter drucklosen Bedingungen substituierte 5-Nitrosopyrimidine in 5-Formylaminopyrimidine umzuwandeln.

Diese Aufgabe wird erfindungsgemäß dadurch gelöst, daß man die Ausgangsverbindungen einer reduktiven Formylierung in Gegenwart von Edelmetallkatalysatoren sowie von Ameisensäure und von einem Formiat unterwirft. Es hat sich überraschenderweise gezeigt, daß man auf diese Weise 5-Formylamino-pyrimidine in hohen Ausbeuten und guten Reinheiten in einem Einstufenverfahren aus den entsprechenden Nitrosoderivaten herstellen kann, ohne daß die Anwendung von Druck und gasförmigem Wasserstoff erforderlich ist.

Beim Verfahren der Erfindung werden als Ausgangsverbindungen 5-Nitrosopyrimidine der allgemeinen Formel (II) 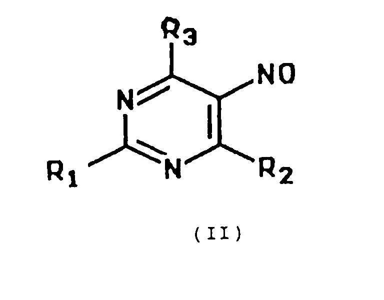
eingesetzt, wobei die Reste R¹ bis R³ gleich oder verschieden sein können und H, OH, SH, NH₂, Alkylamino, Halogen, O-Alkyl, S-Alkyl, Alkyl sowie Aryl bedeuten und Alkyl einen aliphatischen Rest mit 1 bis 4 Kohlenstoffatomen darstellt. Als Arylreste sind Phenylreste bevorzugt, die gegebenenfalls noch substituiert sein können.

Diese Ausgangsverbindungen werden in Wasser und/oder einem organischen Lösemittel suspendiert, wobei als organisches Lösemittel vorzugsweise Formamid und/oder Ameisensäure verwendet wird. Die Ameisensäure kann auch als verdünnte wäßrige Lösung mit einer Konzentration von 20 bis 100 Gew.-% eingesetzt werden. Die Konzentration der Ausgangsverbindung in der Suspension kann in weiten Grenzen variiert werden und beträgt vorzugsweise 0,1 bis 3,0 mol, insbesondere 1,0 bis 2,0 mol Nitrosoverbindung pro 1 Solvens.

Falls nicht in Ameisensäure als Lösemittel gearbeitet wird, wird dieser Suspension Ameisensäure, vorzugsweise in einer Menge von 1,0 bis 10,0 mol, besonders bevorzugt 3,5 bis 5,0 mol pro mol Nitrosoverbindung zugesetzt. Die Zugabe kann vor und während der Reaktion erfolgen.

Als Ameisensäuresalze können bevorzugt Formiate von Alkalimetallen, Erdalkalimetallen, Ammoniak oder Aminen verwendet werden. Besonders bevorzugt sind Natrium- oder Kaliumformiat, Ammoniumformiat oder Trialkylammoniumverbindungen, wie z. B. Triethylammoniumformiat. Der Einsatz der Ammoniumformiate empfiehlt sich besonders dann, wenn die Mutterlaugen verbrannt werden sollen, da in diesem Falle eine rückstandslose Verbrennung möglich ist. Die Formiate werden vorzugsweise in einem Molverhältnis von 0,1 bis 5, bezogen auf die Nitrosoverbindung eingesetzt. Die Zugabe der Formiate kann in fester oder gelöster Form erfolgen.

Außerdem kann das Formiat auch in situ gebildet werden durch Zugabe von z.B. Natronlauge bzw. dem entsprechenden Amin in die Ameisensäure-haltige Suspension. In diesem Fall muß die dafür benötigte Ameisensäure zusätzlich zu der oben genannten Menge vorgelegt werden.

Der Suspension wird ein Edelmetall-Katalysator, beispielsweise auf Basis von Palladium oder Platin, auf einem Trägermaterial zugesetzt, wobei die Menge an zugesetztem Katalysator vom jeweiligen Edelmetallgehalt abhängt. Handelsübliche Katalysatoren haben sich als geeignet erwiesen. Vorzugsweise wird der Katalysator in einer solchen Menge verwendet, daß der Anteil des reinen Edelmetalls 200 bis 2000 mg pro kg eingesetzter Nitrosoverbindung beträgt. Als Trägermaterial für den Edelmetallkatalysator hat sich Aktivkohle bewährt, wobei der Edelmetallgehalt 0,1 bis lO Gew.-% beträgt. Besonders bevorzugt ist die Verwendung eines Katalysators mit etwa 5 Gew.-% Palladiumgehalt auf Aktivkohle insbesondere mit einem Wassergehalt von 40 bis 60 Gew.-%, der in einer Menge von 1 bis 3 Trockengew.-%, bezogen auf die Nitrosoverbindung, eingesetzt wird.

Nach der Zugabe des Katalysators tritt unter Rühren bereits bei Raumtemperatur eine Freisetzung von CO₂ ein. Anschließend wird die Reaktionstemperatur eingestellt, die vorzugsweise zwischen 50 und 200°C, insbesondere 90 bis 11O°C, liegt. Abhängig von den Reaktionsbedingungen bzw. -partnern ist die Reaktion nach 1 bis 10, üblicherweise nach 2 bis 3 Stunden beendet, wobei die vollständige Umsetzung an der Entfärbung der vorher in den meisten Fällen intensiv rot bis violett gefärbten Suspension erkennbar ist.

Für viele Folgereaktionen ist die Abtrennung des Katalysators nach Abschluß der reduktiven Formylierung nicht unbedingt erforderlich, wenn in nachfolgenden Verfahrensschritten die Möglichkeit dazu gegeben ist. Die anfallenden 5-Formylamino-pyrimidine besitzen in der Regel eine so hohe Reinheit, daß sie ohne weitere Reinigungsverfahren zur Synthese von Folgeprodukten, wie z. B. Purinen, eingesetzt werden können.

Falls eine Abtrennung des Katalysators gewünscht wird, empfiehlt es sich, zur Isolierung des Reaktionsproduktes die Suspension zu kühlen, den Feststoff, beispielsweise durch Filtration, abzutrennen und den Filterkuchen mit Wasser nachzuwaschen. Zur Abtrennung des Edelmetallkatalysators wird der Filterkuchen dann in verdünnter, beispielsweise 5 gew.-%iger Natronlauge gelöst, der Katalysator abfiltriert und das Produkt durch Zugabe einer Säure, vorzugsweise Ameisensäure, wieder ausgefällt.

In vielen Fällen ist es auch möglich, durch entsprechende Verdünnung das Produkt in Lösung zu halten. In diesem Fall trennt man zweckmäßig den Katalysator aus der noch heißen Lösung ab und läßt das Produkt durch Einengen der Lösung und/oder Abkühlen auskristallisieren. Auf diese Weise erhält man ein sehr reines Produkt.

Erfindungsgemäß ist es möglich, 5-Formylamino-pyrimidine mit einer Ausbeute von bis zu 98 % und einer Reinheit von mehr als 98 % herzustellen. Aufgrund dieser hohen Ausbeute und des relativ geringen technischen Aufwandes (keine Druckreaktion, keine Verwendung von Wasserstoff) ist das erfindungsgemäße Verfahren besonders gut für die Durchführung im technischen Maßstab geeignet.

Die nachfolgenden Beispiele sollen die Erfindung näher erläutern.

### Beispiele

### Beispiel 1

31,7 g (0,2 mol) 2,4-Diamino-6-hydroxy-5-nitrosopyrimidin (DAHNP) wurden in 100 ml Wasser suspendiert und 37,2 g (0,8 mol) Ameisensäure (98 %-ig) zugegeben. Anschließend wurden 6,4 g (0,1 mol) Ammoniumformiat eingerührt und 1,0 g eines Pd-Katalysators auf Aktivkohle (Typ E10 R/W, 5 % Pd, 50 % feucht) zugesetzt. Schon bei Raumtemperatur setzte eine leichte Gasentwicklung ein. Die Temperatur wurde bis zum Rückfluß auf 1OO°C erhöht und unter gutem Rühren 2 Stunden gehalten. Nach etwa 1,2 Stunden hat sich die anfangs rosafarbene Suspension aufgrund des Katalysatorgehaltes nach grau entfärbt. Anschließend wurde auf 10°C abgekühlt und der Kristallbrei über eine Filternutsche abgesaugt. Der Filterkuchen wurde in 160 ml 5 Gew.-%-iger Natronlauge gelöst und der Edelmetallkatalysator durch Filtration abgetrennt. Durch Zugabe von 9,3 g Ameisensäure (85 Gew.-%-ig) wurde das Produkt wieder ausgefällt, anschließend abfiltriert, zweimal mit je 15 ml Wasser gewaschen und im Vakuum bei 60°C getrocknet.

Auf diese Weise wurden 32,1 g farblose Kristalle mit einem Gehalt von 98,1 % 2,4-Diamino-5-formylamino-6-hydroxypyrimidin erhalten. Dies entspricht einer Ausbeute von 94,8 %.

### Beispiel 2

31,7 g (0,2 mol) DAHNP wurden in 100 ml 85 %-iger Ameisensäure suspendiert. Dazu wurden 44,3 g (0,6 mol) Ammoniak als 23 %-ige wäßrige Lösung zugetropft und 1,0 g Palladiumkatalysator entsprechend Beispiel 1 zugegeben. Unter CO₂-Entwicklung und kräftigem Rühren wurde auf 100°C erhitzt und diese Temperatur 3 Stunden gehalten.

Nach 2 Stunden hatte sich die Suspension entfärbt. Nach dem Abkühlen wurde in einer Probe der Umsatz zu DAFHP mittels HPLC bestimmt. Es wurde ein Gehalt von 32,3 g ermittelt. Dies entspricht einem Umsatz von 96 %, wobei keine Reste der Ausgangsverbindung oder Nebenprodukte nachgewiesen werden konnten.

### Beispiel 3

31,7 g (0,2 mol) DAHNP wurden in 100 ml Formamid suspendiert und 38,4 g (0,6 mol) Ammoniumformiat zugesetzt. Nach Zugabe von 37,2 g (0,8 mol) Ameisensäure wurde 1,0 g Pd-Katalysator entsprechend Beispiel 1 eingerührt und auf 100°C erhitzt. Unter Gasentwicklung entfärbte sich die Suspension innerhalb von 3 Stunden. Nach dem Abkühlen wurde der Umsatz in einer entnommenen Probe mittels HPLC bestimmt. Es ergab sich ein Umsatz von 96,2 % zu DAFHP. Die Ausgangsverbindung oder Nebenprodukte konnten nicht nachgewiesen werden.

### Beispiel 4

Zu einer Suspension von 31,7 g (0,2 mol) DAHNP in 100 ml Wasser wurden 37,2 g (0,8 mol) Ameisensäure und 20,5 g (0,3 mol) Natriumformat gegeben. Nach Zugabe von 1,0 g Pd-Katalysator entsprechend Beispiel 1 wurde zum Rückfluß erhitzt. Dabei wurde unter gutem Rühren CO₂ frei. Nach 2 Stunden hatte sich die Suspension entfärbt und nach einer weiteren Stunde wurde abgekühlt. Durch Filtration wurden 33,6 g graue Kristalle erhalten. Diese wurden in 160 ml 5 %-iger Natronlauge gelöst, der Katalysator abfiltriert und das Produkt durch Zugabe von 9,5 g 98 %-iger Ameisensäure wieder ausgefällt. Nach Kühlen auf O°C wurden 33,0 g (97,8 %) DAFHP als farbloses feinkristallines Pulver erhalten.

### Beispiel 5

31,7 g (0,2 mol) DAHNP wurden in 1500 ml Wasser suspendiert und dann 37,2 g (0,8 mol) Ameisensäure, 38,7 g (0,6 mol) Ammoniumformiat und 1,0 g Pd-Katalysator entsprechend Beispiel 1 zugesetzt. Die Suspension wurde unter kräftigem Rühren zum Rückfluß erhitzt, wobei nach 3 Stunden eine schwarze Lösung entstand. Diese Lösung wurde zur Abtrennung des Katalysators heiß filtriert und langsam abgekühlt. Dabei fielen farblose Kristalle von DAFHP aus. Die Kristallisation wurde durch Kühlen auf O°C vervollständigt und die Kristalle abgesaugt. Es wurden 26,7 g (79 %) farblose Kristalle mit einer Reinheit von 99,1 % erhalten.

### Beispiel 6

Eine Suspension von 7,9 g (0,05 mol) 2,4,6-Triamino-5-nitrosopyrimidin in 50 ml Wasser wurde mit 3,25 g (0,05 mol) Ammoniumformiat, 13,9 g (0,3 mol) Ameisensäure und 0,5 g Pd-Katalysator entsprechend Beispiel 1 versetzt. Diese Mischung wurde 4 Stunden auf 1OO °C erhitzt. Nach dem Abkühlen wurde der Katalysator abfiltriert, das Filtrat auf ca. 25 ml eingeengt und mit ca. 1O ml 25 % Natronlauge bis pH 7,5 neutralisiert. Dabei entstand ein Kristallbrei, der abgesaugt wurde. Auf diese Weise wurden 8,1 g (87 %) hellbeige Kristalle von 2,4,6-Triamino-5-formylamino-pyrimidin-Hydrat erhalten.

| | | | | | |
|---|---|---|---|---|---|
| Ber. | C: | 32,25 | Gef. | C: | 32,19 |
| | H: | 5,41 | | H: | 5,60 |
| | N: | 45,14 | | N: | 44,98 |

### Beispiel 7

15,5 g (0,1 mol) 4,6-Dihydroxy-2-methyl-5-nitrosopyrimidin wurden in 50 ml Wasser suspendiert, 18,2 g (0,4 mol) Ameisensäure und 3,4 g (0,05 mol) Natriumformiat zugegeben und unter Rühren 0,5 g Pd-Katalysator entsprechend Beispiel 1 zugesetzt. Die gefärbte Suspension wurde dann bis zur Entfärbung auf 100°C erhitzt, auf 10°C abgekühlt und die Kristalle abgesaugt. Der Filterkuchen, der noch Katalysator enthielt, wurde aus Wasser unter Heißfiltration umkristallisiert. Die Kristalle wurden im Vakuum bei 60°C getrocknet und in einer Ausbeute von 13,5 g (80 % d. Th.) erhalten.

| | | | | | |
|---|---|---|---|---|---|
| Ber. | C: | 42,60 | Gef. | C: | 42,58 |
| | H: | 4,17 | | H: | 4,29 |
| | N: | 24,84 | | N: | 24,69 |

## Patentansprüche

1. Verfahren zur Herstellung von 5-Formylamino-pyrimidinen der allgemeinen Formel (I), 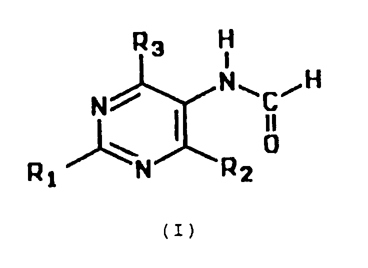 wobei die Reste R¹ bis R³ gleich oder verschieden sind und H, OH, SH, NH₂, Alkylamino, Halogen, O-Alkyl, S-Alkyl, Alkyl sowie Aryl bedeuten und Alkyl einen aliphatischen Rest mit 1 bis 4 Kohlenstoffatomen darstellt, aus den entsprechenden 5-Nitrosopyrimidinen,
**dadurch gekennzeichnet,**
daß man die Ausgangsverbindung einer reduktiven Formylierung in Gegenwart eines Katalysators auf Edelmetallbasis sowie von Ameisensäure und einem Salz davon unterwirft.

2. Verfahren nach Anspruch 1,
**dadurch gekennzeichnet,**
daß man die reduktive Formylierung in Wasser und/oder einem organischen Lösemittel durchführt.

3. Verfahren nach Anspruch 2,
**dadurch gekennzeichnet,**
daß man als organisches Lösemittel Formamid und/oder Ameisensäure verwendet.

4. Verfahren nach einem der Ansprüche 1 bis 3,
**dadurch gekennzeichnet,**
daß man die Konzentration der Nitrosoverbindung auf 0,1 bis 3,0 mol, vorzugsweise 1,0 bis 2,0 mol pro Liter Solvens einstellt.

5. Verfahren nach einem der vorhergehenden Ansprüche,
**dadurch gekennzeichnet,**
daß man als Ausgangsverbindung 2,4-Diamino-6-hydroxy-5-nitrosopyrimidin oder 2,4,6-Triamino-5-nitrosopyrimidin einsetzt.

6. Verfahren nach einem der vorhergehenden Ansprüche,
**dadurch gekennzeichnet,**
daß man die Ameisensäure in einer Menge von 1,0 bis 10,0 mol, vorzugsweise 3,5 bis 5 mol pro mol eingesetztes Nitrosopyrimidin, verwendet.

7. Verfahren nach einem der vorhergehenden Ansprüche,
**dadurch gekennzeichnet,**
daß man als Ameisensäuresalz ein Formiat von Alkalimetallen, Erdalkalimetallen, Ammoniak oder Aminen einsetzt.

8. Verfahren nach einem der vorhergehenden Ansprüche,
**dadurch gekennzeichnet,**
daß man das Formiat in einer Menge von 0,1 bis 5 mol pro mol eingesetztes Nitrosopyrimidin verwendet.

9. Verfahren nach einem der vorhergehenden Ansprüche,
**dadurch gekennzeichnet,**
daß man einen Katalysator auf Basis Palladium oder Platin einsetzt.

10. Verfahren nach einem der vorhergehenden Ansprüche,
**dadurch gekennzeichnet,**
daß die Menge des reinen Edelmetalls 200 bis 2000 mg pro kg eingesetzter Nitrosoverbindung beträgt.

11. Verfahren nach einem der vorhergehenden Ansprüche,
**dadurch gekennzeichnet,**
daß man den Katalysator auf einem Aktivkohle-Trägermaterial einsetzt und der Katalysator einen Edelmetallgehalt 0,1 bis 10 Gew.-% aufweist.

12. Verfahren nach einem der vorhergehenden Ansprüche,
**dadurch gekennzeichnet,**
daß man die Reaktionstemperatur auf 50 bis 200°C, vorzugsweise 90 bis 110°C, einstellt.

13. Verfahren nach einem der vorhergehenden Ansprüche,
**dadurch gekennzeichnet,**
daß man den Katalysator von der Reaktionsmischung abfiltriert und das Produkt anschließend durch Einengen und/oder Abkühlen aus der Lösung auskristallisiert.

14. Verfahren nach einem der vorhergehenden Ansprüche,
**dadurch gekennzeichnet,**
daß man der Reaktionsmischung Natronlauge zusetzt, bis sich das Produkt vollständig gelöst hat, dann den Katalysator vom gelösten Produkt abfiltriert und anschließend mit Säure, vorzugsweise Ameisensäure, die Formylamino-Verbindung wieder ausfällt.
